# EUROPEAN PATENT SPECIFICATION

(11) **EP 4 139 108 B1**
(45) Date of publication and mention of the grant of the patent: **29.05.2024**
(21) Application number: 20721199.6
(22) Date of filing: 22.04.2020
(51) Int. Cl.: B29C 45/14, B29C 43/18, B29C 70/68, B29C 70/70, B29C 70/72, A61L 27/40

(54) **PREPARATION OF A FIBER-REINFORCED IMPLANT**
HERSTELLUNG EINES FASERVERSTÄRKTEN IMPLANTATS
PRÉPARATION D'UN IMPLANT RENFORCÉ PAR DES FIBRES

(43) Date of publication of application: 01.03.2023
(73) Proprietor: Arctic Biomaterials Oy, 33520 Tampere (FI)
(72) Inventor: HUTTUNEN, Mikko, 33520 Tampere (FI); HEINO, Harri, 33520 Tampere (FI); LEHTOLA, Miika, 33520 Tampere (FI); ELLÄ, Ville, 33520 Tampere (FI); LAAKKONEN, Mika, 33520 Tampere (FI)
(74) Representative: Kolster Oy Ab
(86) International application number: PCT/EP2020/061210
(87) International publication number: WO 2021/213648

(56) References cited:
- WO-A1-87/04916
- WO-A1-98/28120
- WO-A1-2014/072983
- US-A- 4 312 917
- US-A- 4 943 404

## Description

### FIELD OF THE INVENTION

The present invention relates to a fiber reinforced implant structure, and to a method for manufacturing the fiber reinforced implant structure. The present invention further relates to an implant comprising the fiber reinforced implant structure.

### BACKGROUND ART

Fibre-reinforced polymer is a composite material made of a polymer matrix reinforced with fibres, commonly used in the aerospace, automotive, marine, and construction industries. The potential use of fiber composite technology in the creation of medical implants may solve certain challenges of currently used metals such as corrosion, disturbance in imaging (such as magnetic resonance imaging, MRI) and mismatch of mechanical properties on between bone and implant material. Compared to polymer implants, fiber reinforced composite implants are able to exhibit superior mechanical properties, improved biocompatibility, etc. The potential of the fiber-reinforced composites in implant technology is based on the fact that these materials exhibit excellent mechanical properties exceeding the mechanical properties of plain polymers and/or particle filled composites. Typically, the improved mechanical properties are the most important reason for using the fiber reinforced composite materials.

Injection molding is a process for manufacturing parts where molten material, such as thermoplastic and/or thermosetting polymer(s) is injected into a mold. The raw material is fed into a heated barrel, mixed using a helical shaped screw, and forced into a mold cavity by injecting. After that the material cools and hardens to the configuration of the mold cavity. High pressure injection of the raw material into the mold shapes the polymer into the desired shape. The sequence of events during the injection molding of the plastic part may be referred to as an injection molding cycle which begins when the mold closes, followed by the injection of the polymer into the mold cavity. Once the cavity is filled, a holding pressure is maintained to prevent material shrinkage. Once the part is cooled down to a desired temperature, the mold opens and the part is ejected. After that the molding cycle to manufacture the next part begins and these molding cycles are repeated until the required amount of products have been manufactured.

Overmolding is an injection molding process where molten matrix material is injected into an injection mold cavity where the separate insert part exists. Said separate insert is placed into the mold cavity prior to the injection of the molten matrix material. The outcome is an overmolded product formed of the insert and matrix material. Overmolding may be also used with other type of molding processes. Overmolding may be also used e.g. in compession molding where a separate insert part is placed in a mold cavity prior to a compression molding cycle. Similarly to the injection molding, the outcome of the compression molding process is an overmolded product formed of the insert and matrix material.

The insert may include e.g. a metallic thread insert, reinforcement fabrics, suture(s), etc. A challenge with fabric and/or other flexible inserts is their movement and/or uncontrolled shape transformation with the polymer melt flow in the mold during the injection of the melt into the mold cavity. This may jeopardise the desired functionality of the insert.

The documents WO 87/04916 A1 and WO 2014/072983 A1 are related to the manufacture of fiber reinforced medical implant structure.

### SUMMARY

The following presents a simplified summary of features disclosed herein to provide a basic understanding of some exemplary aspects of the invention. This summary is not an extensive overview of the invention. It is not intended to identify key/critical elements of the invention or to delineate the scope of the invention. Its sole purpose is to present some concepts disclosed herein in a simplified form as a prelude to a more detailed description.

According to an aspect, there is provided the subject matter of the independent claims. Embodiments are defined in the dependent claims.

One or more examples of implementations are set forth in more detail in the description below. Other features will be apparent from the description, and from the claims.

### BRIEF DESCRIPTION OF THE DRAWINGS

In the following the invention will be described in greater detail by means of preferred embodiments with reference to the attached drawings, in which
Figure 1a illustrates an exemplary mold;
Figure 1b shows an exemplary rigid insert placed on the kernel of the mold cavity;
Figure 2a illustrates an exemplary over molded product produced according to the invention;
Figure 2b illustrates an over molded product produced by using a soft/deformable insert;
Figures 3a, 3b, 4a, 4b, 5a, 5b, 6, 7 and 8 illustrate exemplary over molded products / implants according to the invention;
Figures 9 and 10 show measured test results;
Figure 11a illustrates an exemplary rigid insert placed in the mold cavity according to the invention;
Figure 11b illustrates an exemplary overmolded product produced according to the invention;
Figures 12a-12e illustrate the manufacture of a plate shaped implant structure.

### DETAILED DESCRIPTION OF EMBODIMENTS

The following embodiments are exemplary. Although the specification may refer to "an", "one", or "some" embodiment(s) in several locations, this does not necessarily mean that each such reference is to the same embodiment(s), or that the feature only applies to a single embodiment. Single features of different embodiments may also be combined to provide other embodiments. Furthermore, words "comprising", "containing" and "including" should be understood as not limiting the described embodiments to consist of only those features that have been mentioned and such embodiments may contain also features/structures that have not been specifically mentioned.

The present invention discloses a fiber reinforced implant structure, and a method for producing the fiber reinforced implant structure. In the method, a molded continuous fiber reinforced implant or implant structure is produced by over molding, wherein a continuous fiber containing insert is used as a reinforcement in the implant or implant structure. Continuous fiber containing insert is at least partly over molded by thermoplastic matrix polymer(s). The continuous fiber containing insert is rigid. The rigid fiber containing insert is inserted into a mold cavity in an injection molding machine, after which the mold is closed and thermoplastic matrix material is injected into the mold cavity to perform the injection molding by over molding. After possible setting and/or curing, the part is allowed to be cooled down to a desired temperature following mold opening and ejection of the part. Such a part is usable as (or in) a medical implant and may be manufactured by an industrial process. Alternatively, the rigid fiber containing insert is inserted into a mold cavity in a compression molding machine, after which the mold is closed, and thermoplastic matrix material is pressed into the mold cavity to perform the compression molding by over molding. After possible setting and/or curing, the mold may be cooled down to a desired temperature and the part may be ejected from the mold. Such a part is usable as (or in) a medical implant and may be manufactured by an industrial process.

The fiber containing insert is rigid. The rigid form of the fiber containing insert allows the fiber reinforcement to remain in place in a desired position during the injection (or compression) molding process, i.e. during injection (or compression) of the thermoplastic matrix material into the mold cavity. The mold cavity may be exposed to a pressure of a few thousand bars during the injection (or compression) and/or setting/curing. The possible curing phase may be used to heat treat the molded specimen in order to modify certain properties. These properties include, but are not limited to, increasing the degree of crystallinity which increases certain mechanical properties and may improve thermal stability.

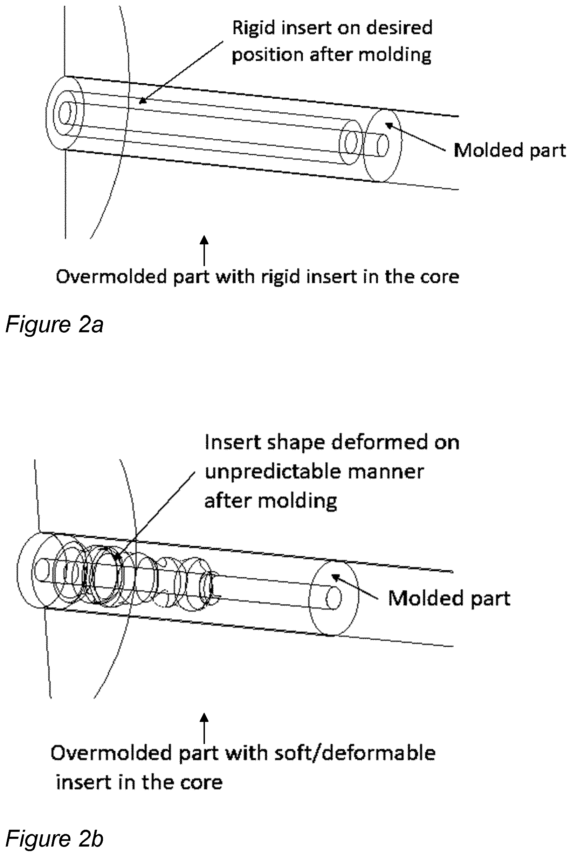

If a soft insert such as an untreated twined insert or untreated knitted insert were used instead of the rigid insert, it would be extremely challenging to hold the insert in place during the injection molding process. If a soft/deformable insert were used as an insert, it would be extremely difficult to ensure that the fibers would be in a desired place/location in the molded part; see Figure 2b illustrating an over molded part with soft/deformable insert in the core.

In an embodiment, the rigid insert may naturally move during the molding process, but the rigid form of the insert enables to tailor the placement of the fiber reinforcement after the molding process. The rigid insert may also be used to tailor the positioning of the reinforcement so that the insert e.g. expands, contracts and/or otherwise moves in connection with the molding to the exact place or shape in which it is desired to fit. For example, the thermoplastic polymer material may be injected into the center of the implant structure, wherein the reinforcement expands to the desired location (onto the surface of the implant).

In an embodiment, the rigid insert contains continuous fibers bound together by using thermoplastic polymer thereby forming a rigid construction. Herein, continuous fibers mean that at least part of the fibers in the insert are as long as the shortest dimension of the product being manufactured, the thinnest wall thickness, or any other dimension easily determined by the geometry of the end product. Preferably, the continuous fibers are at least as long as the longest main dimension, diameter, or other dimension of the article being manufactured.

The fiber length represents the continuity of the fiber. The fibers may break in the process. A continuous fiber is such that it equals to or is longer than the implant's minimum wall thickness. At least some of the fibers in the implant are at least as long as the shortest dimension physically measurable from the implant geometry. If the implant is cylindrical, this means the diameter of the cylinder. In the case of a tube-shaped implant, it means the tube wall thickness. The fiber lenght is thus continuous through a physical dimension of the implant. In reality, the implant may contain fibers, at least some of which are preferably longer than the longest main dimension of the implant. In the case of a cylindrical implant, this means that at least some of the fibers are longer than the diameter of the cylinder. In the case of a tube shaped implant, this means that at least some of the fibers are longer than the wall thickness of the tube. In the Examples below, a "tube-shaped screw" was used, which contained fibers that were considerably longer than the length of the screw. Thus the fibers are considerably longer than the wall thickness, too. As said, a continuous fiber refers to a fiber with a fiber length corresponding to the shortest wall thickness or shortest main dimension of the implant (or molded part).

The rigid continuous fiber containing insert may be produced from thermoplastic polymer-impregnated fiber prepreg, such as impregnated tape, ribbon, wire, etc. The fiber prepreg may comprise any fiber usable as a fiber reinforcement, such as polymeric fiber(s), glass fiber(s), carbon fiber(s), aramid fiber(s), etc. The fiber reinforcement may also be a hybrid fiber reinforcement comprising multiple different reinforcing fibers. The hybrid fiber reinforcement may also contain reinforcing fibers and binding thermoplastic polymer fibers in separate fibers. If such hybrid fiber reinforcement is used, the formed structure is treated, prior to its use as an insert, by molding to melt the thermoplastic polymer fibers to bind the reinforcing fibers and to form a rigid construction.

The thermoplastic matrix material used for the injection (or compression) molding by overmolding may be pure (plain) thermoplastic homopolymer, co-polymer, ter-polymer, a polymer blend, or a mixture of polymer(s) and additive(s), such as a mixture of polymer(s) and calcium phosphate (e.g. beta-tricalcium phosphate (beta-TCP)), a mixture of polymer(s) and glass particles, or a mixture of polymer(s) and chopped glass fiber, chopped carbon fiber and/or any feasible mineral additive, etc.

The insert containing the continuous fiber reinforcement is prepared by a separate process before the molding process. The process for manufacturing the continuous fiber containing insert may be a one-step process or it may include several intermediate steps. In the process of manufacturing the continuous fiber containing insert, a preform (prepreg) comprising fiber reinforcement(s) and thermoplastic polymer impregnant material that binds the fibers, is further processed into a desired shape which is usable as an insert in the final overmolding process. The rigid state of the continuous fiber containing insert is achieved by melting or dissolving the polymer impregnant material that binds the fiber reinforcement(s), whereby the different layers or adjacent structural portions of the polymer impregnant material bond to each other to stiffen the fiber insert structure.

For example, filament winding technology or automatic fiber/tape placement technology may be used for manufacturing the continuous fiber containing insert, thereby providing a desired fiber orientation of the insert and producing multiple layers of fibrous structures with each layer having exactly the desired orientation.

The process for manufacturing the continuous fiber containing insert may also include any manufacturing method(s) used in the textile industry (such as braiding, knitting, weaving, etc.). However, if a common manufacturing process from the textile technology is used, the resulting structure is generally not rigid, and therefore the fiber reinforcement structure thus obtained has to be stiffened in a further process step, whereby the preform is processed e.g. by heat and/or solvent to achieve the rigid structure of the insert.

The process for manufacturing the continuous fiber containing insert may also include 3D printing. However, in that case the entire structure of the insert is not produced by 3D printing.

The process for manufacturing the rigid continuous fiber containing insert may also be a combination of the different manufacturing methods described above, including filament winding, automatic tape placement, 3D printing, solvent casting, vacuum bagging, extrusion, injection molding, compression molding, textile industry processes (such as braiding, knitting, weaving), etc.

The geometry of the part is achieved in the over molding process. In the over molding process, the continuous fiber reinforced insert is placed in the mold cavity, thereafter the mold is closed, and the mold cavity is filled with thermoplastic material to provide the geometry of the final fiber reinforced part. The continuous fiber containing insert may be located in the core of the end product (as in the Example 1 below), inside the end product, or on the surface(s) of the end product (as in the Example 2 below). The fiber reinforcement may also be located on one or more of the surfaces of the end product. There are no geometric constraints on the shape of the end product, other than those imposed on by the molding process. The part shape or the implant shape may thus be of any possible moldable shape, e.g. a plate, screw, nail, etc.

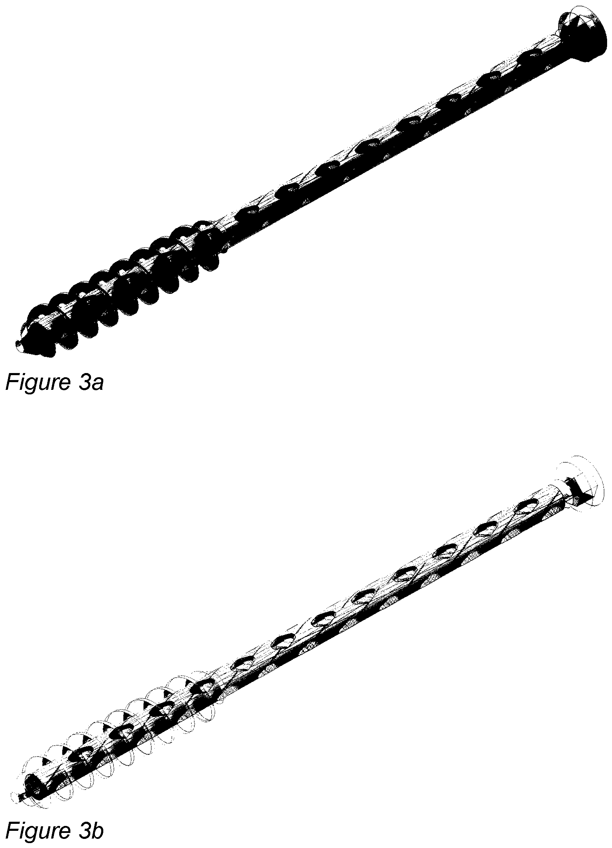

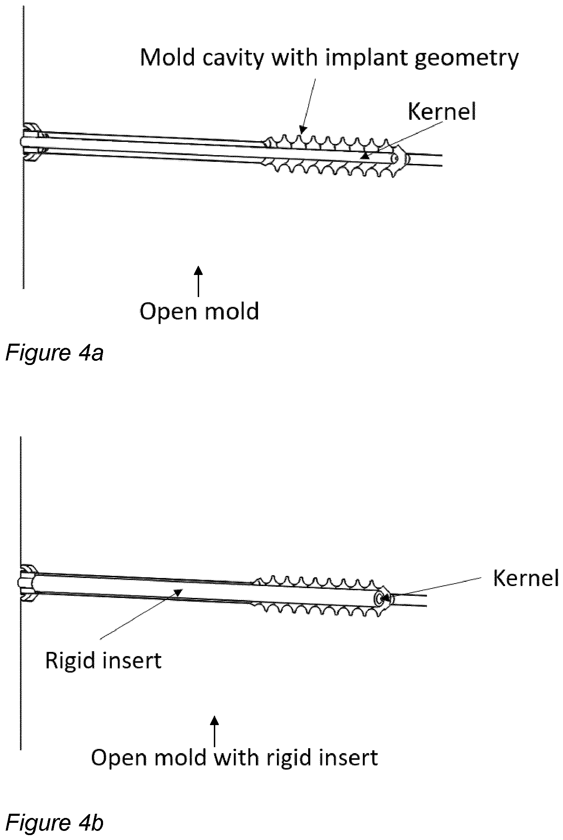

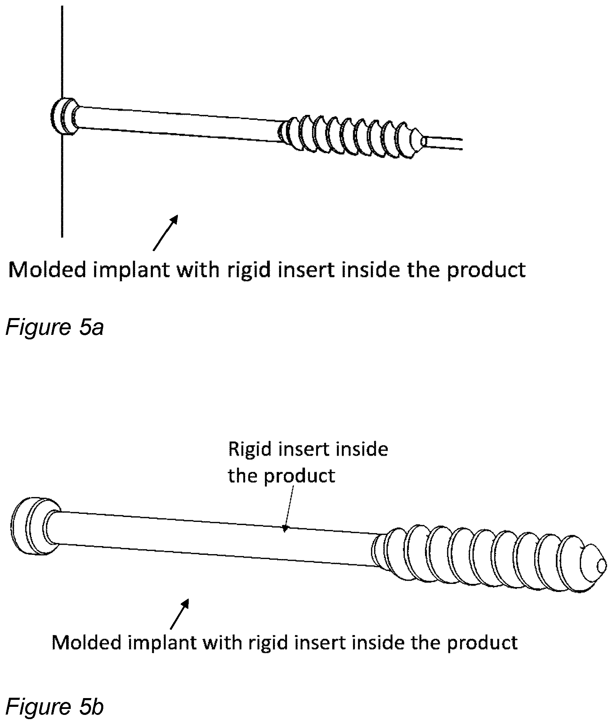

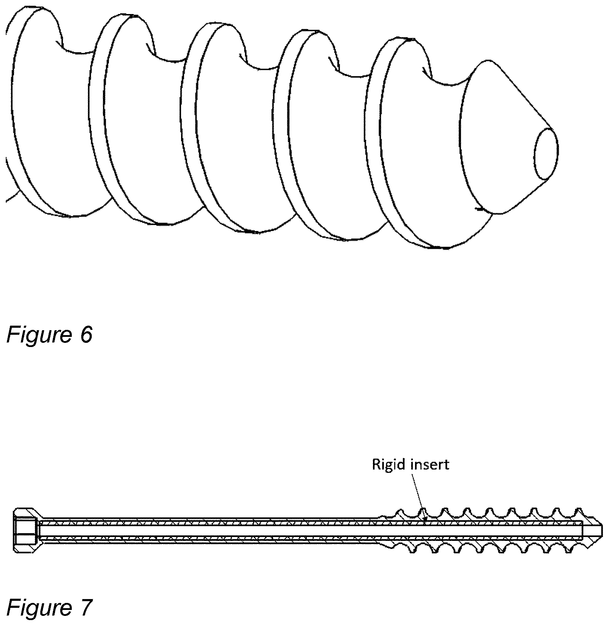

Figures 2a, 3a, 3b, 4a, 4b, 5a, 5b, 6, 7 and 8 illustrate exemplary over molded parts / implants according to the invention. As can be seen from Figures 2a, 3a, 3b, 4a, 4b, 5a, 5b, 6, 7 and 8, the product form of the final product may be any shape which is obtainable by using the present invention. The continuous fiber reinforced insert may be located in the core, surface(s), inside, end or ends of the product, or the location of the continuous fiber containing insert may be any combination of the above locations.

The present invention enables preparing medical implants that solve challenges of certain metals such as corrosion, disturbance in imaging (such as MRI), and mismatch of mechanical properties in between bone and implant material. When compared to polymer implants or other composite materials, the fiber reinforced composite implants are able to exhibit superior mechanical properties, improved cytotoxicity, improved biocompatibility, etc. The potential of fiber-reinforced composites in implant technology is based on the fact that these materials exhibit excellent mechanical properties exceeding the mechanical properties of plain polymers and particle filled composite materials. Typically, the improved mechanical properties are the most important reason for using composite materials. The present invention does not set any limitations for fiber orientation, as the fiber insert may be prepared using manufacturing technique combining multiple manufacturing processes.

Thus an embodiment discloses a structure and a method for manufacturing a fiber-reinforced implant structure. In the method, a fiber-reinforced rigid insert is provided. The insert comprises continuous fibers impregnated with at least one first thermoplastic polymer. A molding cycle is performed by over molding using the pre-manufactured fiber insert, the molding cycle comprising placing the fiber-reinforced rigid insert into a mold cavity, injection or compression molding into the mold cavity at least one second thermoplastic polymer in melted form, such that the fiber-reinforced rigid insert placed into the mold cavity is at least partly covered by the at least one second thermoplastic polymer, and subjecting the at least one second thermoplastic polymer injection or compression molded into the mold cavity to setting and/or curing, thereby obtaining a molded fiber-reinforced implant structure containing the fiber-reinforced rigid insert at least partly covered by the at least one second thermoplastic polymer. The fiber-reinforced rigid insert is hold still in the mold cavity during the injection molding/compression molding and during the setting/curing or cooling. The at least one first thermoplastic polymer and the at least one second thermoplastic polymer are the same or different.

In an embodiment, the fiber reinforced rigid insert is placed into the mold cavity before the injection or compression molding.

In an embodiment, after placing the fiber reinforced rigid insert into the mold cavity, the mold cavity is sealed and filled with the at least one second thermoplastic polymer.

In an embodiment, the mold cavity is exposed to a pressure of 1 bar to 2500 bar during the injection molding or compression molding.

In an embodiment, the mold cavity is exposed to a pressure of 1 bar to 2500 bar during a back pressure phase.

In an embodiment, the mold cavity is exposed to a pressure of 1 bar to 2500 bar during the setting and/or curing.

In an embodiment, the at least one second thermoplastic polymer comprises pure (plain) thermoplastic polymer, or a polymer blend, and/or the at least one second thermoplastic polymer further contains additive(s).

In an embodiment, the at least one second thermoplastic polymer comprises homopolymer(s), copolymer(s) and/or terpolymer(s).

In an embodiment, the additive is at least one of calcium phosphate(s), such as beta-tricalcium phosphate (beta-TCP), glass particles, chopped fiber glass, chopped carbon fiber, any feasible mineral additive, plasticiser, and nucleating agent. The additive may be any additive usable to modify properties of polymers.

In an embodiment, the fiber reinforced insert comprises polymeric fiber(s), glass fiber(s), carbon fiber(s), and/or aramid fiber(s) impregnated with said at least one first thermoplastic polymer.

In an embodiment, the fiber reinforced insert is prepared by processing an preform comprising fibers impregnated with said at least one first thermoplastic polymer, into a desired shape, and melting and/or dissolving said at least one first thermoplastic polymer, wherein the successive layers and/or adjacent structural portions of said at least one first thermoplastic polymer material bond to each other thereby providing the fiber reinforced rigid insert.

In an embodiment, the fiber reinforced insert is prepared by using filament winding and/or automatic tape/fiber placement.

In an embodiment, the fiber reinforced insert is prepared by using braiding, weaving, and/or knitting, followed by a treatment by heat and/or solvent thereby providing of the fiber reinforced rigid insert.

In an embodiment, the fiber reinforced insert is partially prepared by using 3D printing.

In an embodiment, the fiber reinforced insert may be prepared by using a combined manufacturing method where a free fiber orientation design created by 3D printing is combined with consistent fiber reinforcement created by using another technique. As a non-limiting example of combined manufacturing technique, the core of rigid fiber insert may be formed of filament winded continuous fiber reinforced tube having varying fiber orientation in between the superimposed tube layers (e.g....-45°/45°/-45°/45°... or any other possible fiber orientation obtained by using filament winding) and 3D printed continuous filament which is 3D printed in desired fiber paths on top, in the core or in the middle/in between the filament winded tube. The resulting hybrid insert is rigid both on the core (rigid filament winded tube) and on the structures 3D printed on top of the core. 3D fiber printing enables to align the fiber reinforcement totally freely without restrictions set by conventional manufacturing methods such as filament winding.

In an embodiment, the fibers are at least as long as the shortest dimension of the molded fiber-reinforced implant or implant structure, the thinnest wall thickness, or any other dimension determined by the geometry of the molded fiber-reinforced implant or implant structure, preferably the fibers are at least as long as the longest main dimension, diameter, or other dimension of the molded fiber-reinforced implant or implant structure.

Another embodiment discloses a molded fiber-reinforced implant or implant structure comprising a fiber-reinforced rigid insert. The fiber-reinforced rigid insert includes continuous fibers that are impregnated with at least one first thermo-plastic polymer, and at least one second thermoplastic polymer, such that the fiber-reinforced rigid insert is at least partly covered by over molding by the at least one second thermoplastic polymer in the molded fiber-reinforced implant or implant structure. The at least one first thermoplastic polymer and the at least one second thermoplastic polymer are the same or different.

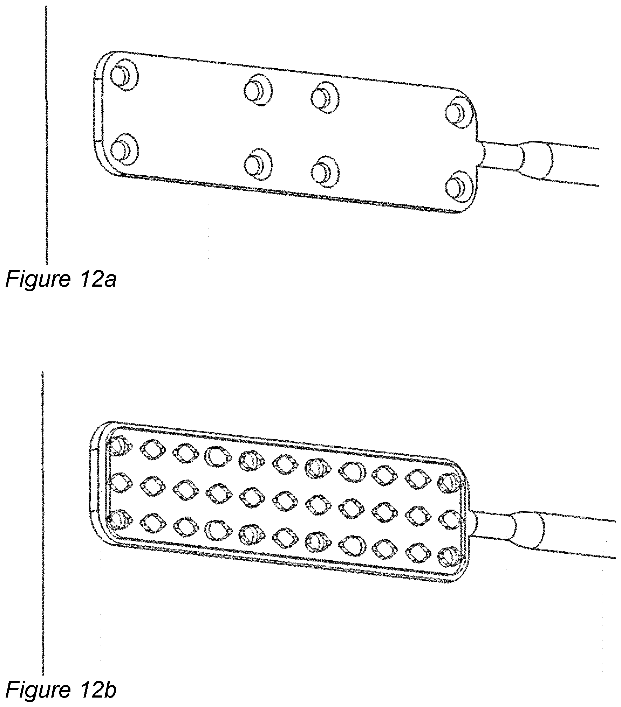

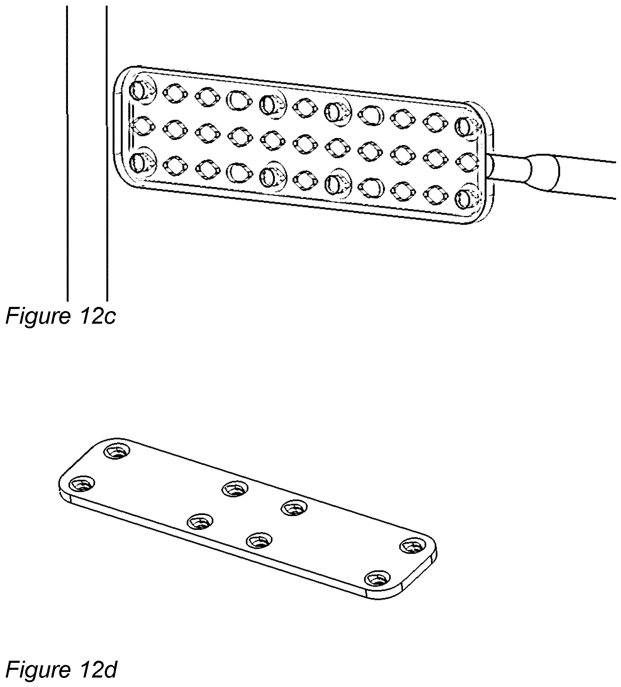

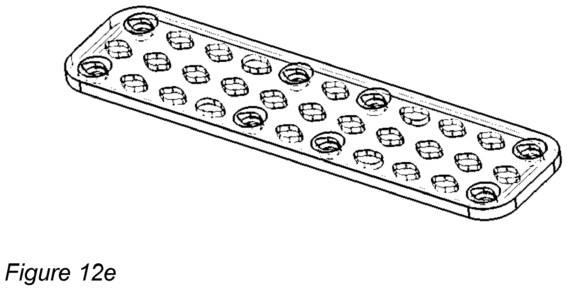

In an embodiment, the implant or implant structure has a shape of a plate, screw or nail. Figures 12a-12e illustrate the manufacture of a plate shaped implant structure.

In an embodiment, the implant or implant structure may be hollow or solid.

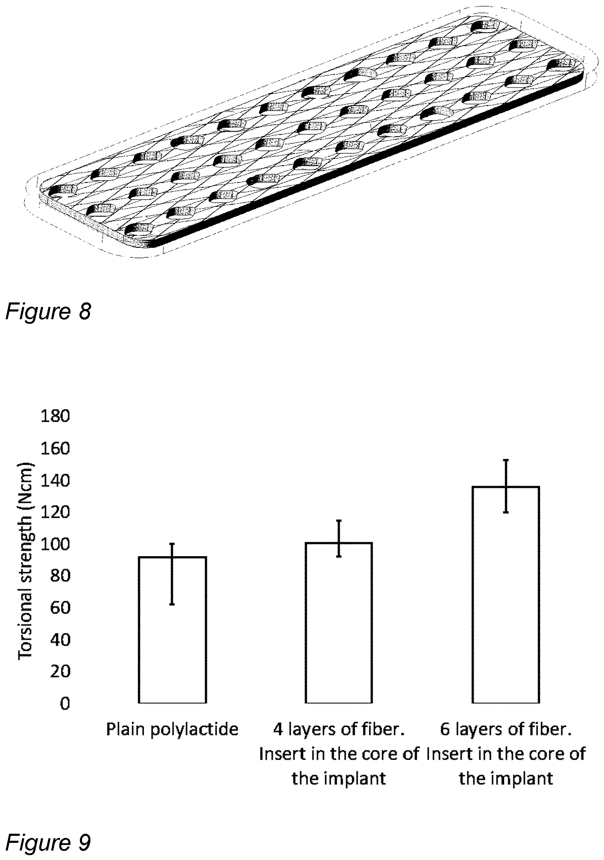

In an embodiment, the implant or implant structure may be perforated (see Figure 8).

In an embodiment, the insert may be hollow or solid.

In an embodiment, the insert may be perforated (see Figure 8).

In an embodiment, the implant or implant structure comprises polymeric fiber(s), glass fiber(s), carbon fiber(s), and/or aramid fiber(s).

In an embodiment, the insert comprises multiple layers of continuous fibers, with each layer being in a selected fiber orientation in the insert.

In an embodiment, the insert is located in the core of the implant or implant structure, inside the implant or implant structure, or at least partly on a surface of the implant or implant structure.

In an embodiment, the implant or implant structure is produced by the method as described above.

In an embodiment, a medical implant is provided, comprising the implant structure as described above.

### EXAMPLE 1

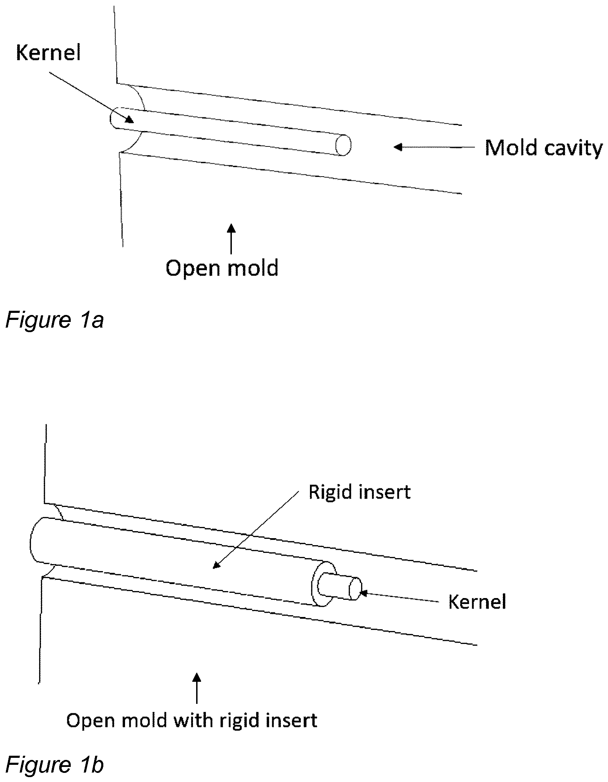

A rigid fiber reinforced insert was prepared by using filament winding as the fabrication technique, in which the preformed tape including continuous glass fibers and thermoplastic polymer matrix, was further processed into a four and six layer tubular structures. During the filament winding the preform tape was heated above the melting point of binding thermoplastic polymer. The fiber orientation in the layers of the four layer construction was 45° / -45° / 45° / -45° / .... After that the preformed rigid insert was placed into a mold cavity on top of the core (see Figure 1b). Thermoplastic matrix material was then injection molded onto the fiber reinforced insert in the mold cavity, wherein a fiber-reinforced implant was obtained as the final injection molded part (see Figure 2a). In Example 1, the fiber reinforcement was located in the core of the implant. Torsional strength of the manufactured composites were compared to an identical product design composed of plain polymer. Results are shown in Figure 9.

### EXAMPLE 2

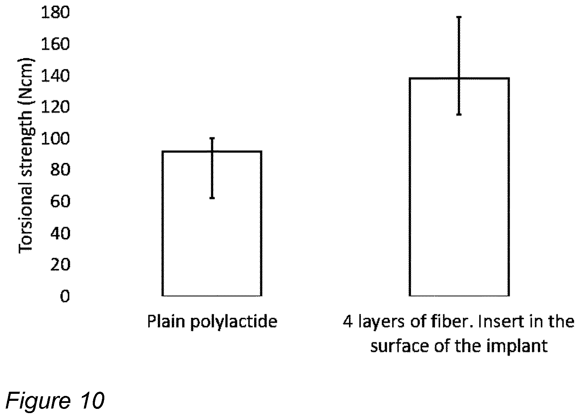

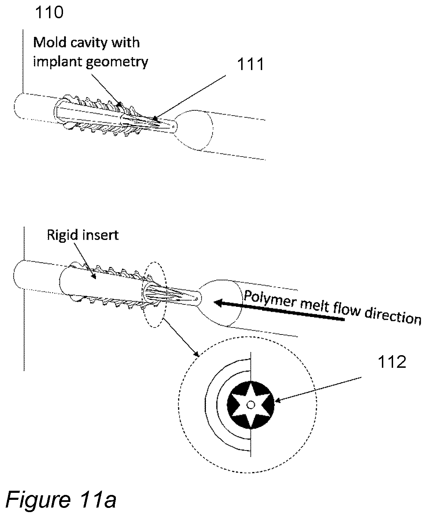

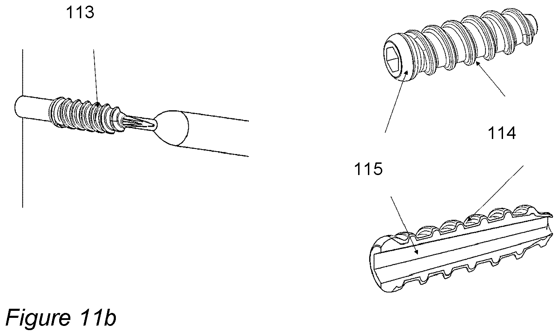

A rigid fiber reinforced insert was prepared by using filament winding as the fabrication technique. A preformed tape including continuous glass fibers and thermoplastic polymer matrix was further processed into a four layer tubular structure. During the filament winding the preform tape was heated above the melting point of binding thermoplastic polymer. The fiber orientation in the layers of four layer construction was 45° / -45° / 45° / -45°. After that the preformed rigid insert was placed into a mold cavity on top of the core pin (see Figure 11a). The insert was designed so that injected matrix polymer filled the mold from the core of the implant and from the core of the rigid insert. Thermoplastic matrix material was then injection molded into the mold cavity, wherein a fiber-reinforced implant was obtained as the final injection molded part (see Figure 11b). The rigid insert was designed so that the injected material flowed into the middle of mold, inside the tube, which forced the tubular insert to expand slightly towards the mold walls. Despite of the fact that the used tubular insert was rigid, a controlled expansion was possible, as the thermoplastic polymer binding the glass fiber layers of the rigid insert was melted simultaneously, while the melted matrix polymer was injected into the mold cavity with a 1500 bar pressure. Thus the rigid insert enabled a controlled movement of the insert during molding and allowed for tailoring the location of the reinforcing fibers in the end product. In Example 2, the fiber reinforcement was located on the surface of the implant. Torsional strength of the manufactured composites wes compared to an identical product design composed of plain polymer. Results are shown in Figure 10.

### EXAMPLE 3

Figures 11a - 11b show an exemplary situation where the reinforcement was placed in the core, the thermoplastic polymer material was injected to the center of the mold, after which the reinforcement expanded in a controlled manner towards the surface, see item 114. Only with a rigid reinforcement such controlled expansion was possible. If a non rigid insert were used, the situation after molding would be similar to that presented in Figure 2b. In Figure 11b, item 114 illustrates the rigid insert after molding, having a tailored position in the implant structure. In Figure 11b, item 113 illustrates the outcome after molding where the molded implant with the rigid insert expanded towards the outer mold surface. In Figure 11b, item 115 illustrates the molded core or matrix injected into the mold. In Figure 11a, item 112 illustrates a space which allows the polymer melt to flow to the inside of the specimen, where the pressure caused by the melt flow expands the initially rigid insert towards the mold walls. In Figure 11a, item 111 shows a specially shaped kernel to enable mold filling from the center of the core.

It will be obvious to a person skilled in the art that, as the technology advances, the inventive concept can be implemented in various ways. The invention and its embodiments are not limited to the examples described above but may vary within the scope of the claims.

## Claims

1. A method for manufacturing a fiber-reinforced medical implant structure,
providing a fiber-reinforced rigid insert comprising continuous fibers impregnated with at least one first thermoplastic polymer,
performing a molding cycle by overmolding, comprising
placing the fiber-reinforced rigid insert into a mold cavity;
injection or compression molding into the mold cavity at least one second thermoplastic polymer in melted form, such that the fiber-reinforced rigid insert placed into the mold cavity is at least partly covered by the at least one second thermoplastic polymer, and
cooling the at least one second thermoplastic polymer injection or compression molded into the mold cavity, thereby obtaining a molded fiber-reinforced medical implant structure containing the fiber-reinforced rigid insert at least partly covered by the at least one second thermoplastic polymer,
wherein the fiber-reinforced rigid insert is in a predefined location in the mold cavity during said injection or compression molding and during said cooling,
wherein the at least one first thermoplastic polymer and the at least one second thermoplastic polymer are the same or different,
and **characterized in that**
the at least one second thermoplastic polymer further contains additive(s), wherein the additive is at least one of calcium phosphate, beta-tricalcium phosphate (beta-TCP), glass particles, and mineral additive(s).

2. A method as claimed in claim 1,
wherein the fiber reinforced rigid insert is placed into the mold cavity before said injection or compression molding;
wherein after placing the fiber reinforced rigid insert into the mold cavity, the mold cavity is closed and filled with the at least one second thermoplastic polymer; and/or
wherein the mold cavity is exposed to a pressure of 1 bar to 2500 bar during said injection or compression molding and during said cooling.

3. A method as claimed in any of claims 1 to 2, wherein
the at least one second thermoplastic polymer comprises plain thermoplastic polymer, or a polymer blend,
the at least one second thermoplastic polymer comprises homopolymer(s), copolymer(s) and/or terpolymer(s), and/or
the at least one second thermoplastic polymer further contains chopped carbon fibers and/or chopped fiber glass, as additive(s).

4. A method as claimed in any of claims 1 to 3, wherein the fiber reinforced insert comprises polymeric fiber(s), glass fiber(s), carbon fiber(s), and/or aramid fiber(s) impregnated with said at least one first thermoplastic polymer.

5. A method as claimed in any of claims 1 to 4, wherein the fiber reinforced insert is prepared by
processing an preform comprising fibers impregnated with said at least one first thermoplastic polymer, into a desired shape, and
melting or dissolving said at least one first thermoplastic polymer, wherein the successive layers and/or adjacent structural portions of said at least one first thermoplastic polymer material bond to each other thereby providing of the fiber reinforced rigid insert.

6. A method as claimed in any of claims 1 to 5,
wherein the fiber reinforced insert is prepared by using filament winding, automatic tape placement, and/or automatic fiber placement; and/or
wherein the fiber reinforced insert is prepared by using braiding, weaving and/or knitting, followed by a treatment by heat and/or solvent thereby providing the fiber reinforced rigid insert.

7. A method as claimed in claim 6, wherein the fiber reinforced insert is prepared by 3D printing of continuous fibers, and/or by other process for preparing a rigid insert from pre-impregnated material containing continuous fibers.

8. A method as claimed in any of claims 1 to 7, wherein the fibers are at least as long as the shortest dimension of the molded fiber-reinforced medical implant structure, the thinnest wall thickness, or any other dimension determined by the geometry of the molded fiber-reinforced medical implant structure, preferably the fibers are at least as long as the longest main dimension, diameter, or other dimension of the molded fiber-reinforced medical implant structure.

9. A molded fiber-reinforced medical implant structure, comprising
a fiber-reinforced rigid insert, wherein the fiber-reinforced rigid insert includes continuous fibers that are impregnated with at least one first thermoplastic polymer, and
at least one second thermoplastic polymer, such that the fiber-reinforced rigid insert is at least partly covered by over-molding by the at least one second thermoplastic polymer in the molded fiber-reinforced medical implant structure,
wherein the at least one first thermoplastic polymer and the at least one second thermoplastic polymer are the same or different,
and **characterized in that**
the at least one second thermoplastic polymer further contains additive(s), wherein the additive is at least one of calcium phosphate, beta-tricalcium phosphate (beta-TCP), glass particles, and mineral additive(s).

10. A medical implant structure as claimed in claim 9, wherein it has a shape of a plate, screw or nail.

11. A medical implant structure as claimed in claim 9 or 10, wherein
the at least one second thermoplastic polymer comprises plain thermoplastic polymer, or a polymer blend,
the at least one second thermoplastic polymer comprises homopolymer(s), copolymer(s) and/or terpolymer(s), and/or
the at least one second thermoplastic polymer further contains chopped carbon fibers and/or chopped fiber glass, as additive(s).

12. A medical implant structure as claimed in any of claims 9 to 11, wherein it comprises polymeric fiber(s), glass fiber(s), carbon fiber(s), and/or aramid fiber(s) impregnated with the at least one first thermoplastic polymer;
wherein the insert comprises multiple layers of continuous fibers, with each layer being in a selected fiber orientation in the insert; and/or
wherein the insert comprises multiple layers of continuous fibers, with each layer being in a selected fiber orientation in the insert and a 3D printed construction 3D printed on top of the layered insert.

13. A medical implant structure as claimed in any of claims 9 to 12, wherein the insert comprises multiple layers of continuous fibers, with each layer being in a selected fiber orientation in the insert and a 3D printed construction 3D printed on the core of the layered insert.

14. A medical implant structure as claimed in any of claims 9 to 13, wherein the fibers are at least as long as the shortest dimension of the molded fiber-reinforced medical implant structure, the thinnest wall thickness, or any other dimension determined by the geometry of the molded fiber-reinforced medical implant structure, preferably the fibers are at least as long as the longest main dimension, diameter, or other dimension of the molded fiber-reinforced medical implant structure.

15. A medical implant structure as claimed in any of claims 9 to 14, wherein the insert is located in the core of the medical implant structure, inside the medical implant structure, or at least partly on a surface of the medical implant structure.

16. A medical implant structure as claimed in any of claims 9 to 15, wherein it is produced by the method of any of claims 1 to 8.

17. A medical implant comprising the medical implant structure as claimed in any of claims 9 to 16.

## Patentansprüche

1. Verfahren zur Herstellung einer faserverstärkten medizinischen Implantatstruktur,
Bereitstellen eines faserverstärkten starren Einlegers, der Endlosfasern umfasst, die mit mindestens einem ersten thermoplastischen Polymer imprägniert sind,
Durchführen eines Formgebungszyklus durch Umspritzen, umfassend:
Einbringen des faserverstärkten starren Einlegers in einen Formhohlraum;
Spritzgießen oder Formpressen mindestens eines zweiten thermoplastischen Polymers in geschmolzener Form in den Formhohlraum, so dass der in den Formhohlraum eingebrachte faserverstärkte starre Einleger zumindest teilweise von dem mindestens einen zweiten thermoplastischen Polymer bedeckt ist, und
Abkühlen des mindestens einen zweiten thermoplastischen Polymers, das in den Formhohlraum spritzgegossen oder formgepresst wurde, wodurch eine geformte faserverstärkte medizinische Implantatstruktur erhalten wird, die den faserverstärkten starren Einleger enthält, der zumindest teilweise von dem mindestens einen zweiten thermoplastischen Polymer bedeckt ist,
wobei sich der faserverstärkte starre Einleger während des Spritzgießens oder Formpressens und während des Abkühlens an einer vordefinierten Stelle in dem Formhohlraum befindet,
wobei das mindestens eine erste thermoplastische Polymer und das mindestens eine zweite thermoplastische Polymer gleich oder verschieden sind,
und **dadurch gekennzeichnet, dass**
das mindestens eine zweite thermoplastische Polymer weiter Additiv(e) enthält, wobei das Additiv mindestens entweder Calciumphosphat, Beta-Tricalciumphosphat (Beta-TCP), Glaspartikel oder mineralische Additiv(e) ist.

2. Verfahren nach Anspruch 1,
wobei der faserverstärkte starre Einleger vor dem Spritzgießen oder Formpressen in den Formhohlraum eingebracht wird;
wobei nach dem Einbringen des faserverstärkten starren Einlegers in den Formhohlraum der Formhohlraum geschlossen und mit dem mindestens einen zweiten thermoplastischen Polymer gefüllt wird; und/oder
wobei der Formhohlraum während des Spritzgießens oder Formpressens und während des Abkühlens einem Druck von 1 bar bis 2500 bar ausgesetzt ist.

3. Verfahren nach einem der Ansprüche 1 bis 2, wobei
das mindestens eine zweite thermoplastische Polymer ein einfaches thermoplastisches Polymer oder eine Polymermischung umfasst,
das mindestens eine zweite thermoplastische Polymer Homopolymer(e), Copolymer(e) und/oder Terpolymer(e) umfasst und/oder
das mindestens eine zweite thermoplastische Polymer weiter geschnittene Kohlenstofffasern und/oder geschnittene Glasfasern als Additiv(e) enthält.

4. Verfahren nach einem der Ansprüche 1 bis 3, wobei der faserverstärkte Einleger Polymerfaser(n), Glasfaser(n), Kohlenstofffaser(n) und/oder Aramidfaser(n) umfasst, die mit dem mindestens einen ersten thermoplastischen Polymer imprägniert sind.

5. Verfahren nach einem der Ansprüche 1 bis 4, wobei der faserverstärkte Einleger hergestellt wird durch
Verarbeiten eines Vorformlings aus mit dem mindestens einen ersten thermoplastischen Polymer imprägnierten Fasern in eine gewünschte Form, und
Schmelzen oder Auflösen des mindestens einen ersten thermoplastischen Polymers, wobei sich die aufeinanderfolgenden Schichten und/oder benachbarten Strukturabschnitte des mindestens einen ersten thermoplastischen Polymermaterials miteinander verbinden, wodurch der faserverstärkte starre Einleger entsteht.

6. Verfahren nach einem der Ansprüche 1 bis 5,
wobei der faserverstärkte Einleger unter Verwendung von Filament Winding, Automated Tape Placement und/oder Automated Fiber Placement hergestellt wird; und/oder
wobei der faserverstärkte Einleger unter Verwendung von Flechten, Weben und/oder Stricken hergestellt wird, gefolgt von einer Behandlung durch Wärme und/oder Lösungsmittel, wodurch der faserverstärkte starre Einleger entsteht.

7. Verfahren nach Anspruch 6, wobei der faserverstärkte Einleger durch 3D-Drucken von Endlosfasern und/oder durch ein anderes Verfahren zur Herstellung eines starren Einlegers aus vorimprägniertem Material, das Endlosfasern enthält, hergestellt wird.

8. Verfahren nach einem der Ansprüche 1 bis 7, wobei die Fasern mindestens so lang sind wie die kürzeste Abmessung der geformten faserverstärkten medizinischen Implantatstruktur, die dünnste Wanddicke oder eine andere Abmessung, die durch die Geometrie der geformten faserverstärkten medizinischen Implantatstruktur bestimmt wird, vorzugsweise sind die Fasern mindestens so lang wie die längste Hauptabmessung, der Durchmesser oder eine andere Abmessung der geformten faserverstärkten medizinischen Implantatstruktur.

9. Geformte faserverstärkte medizinische Implantatstruktur umfassend:
einen faserverstärkten starren Einleger, wobei der faserverstärkte starre Einleger Endlosfasern enthält, die mit mindestens einem ersten thermoplastischen Polymer imprägniert sind, und
mindestens ein zweites thermoplastisches Polymer, so dass der faserverstärkte starre Einleger zumindest teilweise durch Umspritzen mit dem mindestens einen zweiten thermoplastischen Polymer in der geformten faserverstärkten medizinischen Implantatstruktur bedeckt ist,
wobei das mindestens eine erste thermoplastische Polymer und das mindestens eine zweite thermoplastische Polymer gleich oder verschieden sind,
und **dadurch gekennzeichnet, dass**
das mindestens eine zweite thermoplastische Polymer weiter Additiv(e) enthält, wobei das Additiv mindestens entweder Calciumphosphat, Beta-Tricalciumphosphat (Beta-TCP), Glaspartikel oder mineralische Additiv(e) ist.

10. Medizinische Implantatstruktur nach Anspruch 9, wobei sie die Form einer Platte, einer Schraube oder eines Nagels aufweist.

11. Medizinische Implantatstruktur nach Anspruch 9 oder 10, wobei
das mindestens eine zweite thermoplastische Polymer ein einfaches thermoplastisches Polymer oder eine Polymermischung umfasst,
das mindestens eine zweite thermoplastische Polymer Homopolymer(e), Copolymer(e) und/oder Terpolymer(e) umfasst, und/oder
das mindestens eine zweite thermoplastische Polymer weiter geschnittene Kohlenstofffasern und/oder geschnittene Glasfasern als Additiv(e) enthält.

12. Medizinische Implantatstruktur nach einem der Ansprüche 9 bis 11,
wobei sie Polymerfaser(n), Glasfaser(n), Kohlenstofffaser(n) und/oder Aramidfaser(n) umfasst, die mit dem mindestens einen ersten thermoplastischen Polymer imprägniert sind;
wobei der Einleger mehrere Schichten aus Endlosfasern umfasst, wobei jede Schicht in einer ausgewählten Faserorientierung in dem Einleger vorliegt; und/oder
wobei der Einleger mehrere Schichten aus Endlosfasern umfasst, wobei jede Schicht in einer ausgewählten Faserorientierung in dem Einleger vorliegt und eine 3D-gedruckte Konstruktion 3D-gedruckt auf der Oberseite des geschichteten Einlegers ist.

13. Medizinische Implantatstruktur nach einem der Ansprüche 9 bis 12, wobei der Einleger mehrere Schichten aus Endlosfasern umfasst, wobei jede Schicht in einer ausgewählten Faserorientierung in dem Einleger vorliegt und eine 3D-gedruckte Konstruktion 3D-gedruckt auf den Kern des geschichteten Einlegers ist.

14. Medizinische Implantatstruktur nach einem der Ansprüche 9 bis 13, wobei die Fasern mindestens so lang sind wie die kürzeste Abmessung der geformten faserverstärkten medizinischen Implantatstruktur, die dünnste Wanddicke oder eine andere Abmessung, die durch die Geometrie der geformten faserverstärkten medizinischen Implantatstruktur bestimmt wird, vorzugsweise sind die Fasern mindestens so lang wie die längste Hauptabmessung, der Durchmesser oder eine andere Abmessung der geformten faserverstärkten medizinischen Implantatstruktur.

15. Medizinische Implantatstruktur nach einem der Ansprüche 9 bis 14, wobei sich der Einleger im Kern der medizinischen Implantatstruktur, im Innern der medizinischen Implantatstruktur oder zumindest teilweise auf einer Oberfläche der medizinischen Implantatstruktur befindet.

16. Medizinische Implantatstruktur nach einem der Ansprüche 9 bis 15, wobei sie mit dem Verfahren nach einem der Ansprüche 1 bis 8 hergestellt wird.

17. Medizinisches Implantat umfassend die medizinische Implantatstruktur nach einem der Ansprüche 9 bis 16.

## Revendications

1. Procédé pour fabriquer une structure d'implant médical renforcé par des fibres, comprenant
la fourniture d'un insert rigide renforcé par des fibres comprenant des fibres continues imprégnées avec au moins un premier polymère thermoplastique,
la mise en œuvre d'un cycle de moulage par surmoulage, comprenant
le placement de l'insert rigide renforcé par des fibres dans une cavité de moule ;
le moulage par injection ou compression dans la cavité de moule d'au moins un deuxième polymère thermoplastique sous forme fondue, de façon que l'insert rigide renforcé par des fibres placé dans la cavité de moule soit au moins partiellement recouvert par l'au moins un deuxième polymère thermoplastique, et
le refroidissement de l'au moins un deuxième polymère thermoplastique moulé par injection ou compression dans la cavité de moule, ce qui donne ainsi une structure d'implant médical renforcé par des fibres moulé contenant l'insert rigide renforcé par des fibres au moins partiellement recouvert par l'au moins un deuxième polymère thermoplastique,
dans lequel l'insert rigide renforcé par des fibres est en un emplacement prédéfini dans la cavité de moule durant ledit moulage par injection ou compression et durant ledit refroidissement,
dans lequel l'au moins un premier polymère thermoplastique et l'au moins un deuxième polymère thermoplastique sont identiques ou différents,
et **caractérisé en ce que** ledit au moins un deuxième polymère thermoplastique contient en outre un ou plusieurs additifs, dans lequel l'additif est au moins l'un parmi le phosphate de calcium, le bêta-phosphate tricalcique (bêta-TCP), les particules de verre, et un ou plusieurs additifs minéraux.

2. Procédé selon la revendication 1,
dans lequel l'insert rigide renforcé par des fibres est placé dans la cavité de moule avant ledit moulage par injection ou compression ;
dans lequel, après le placement de l'insert rigide renforcé par des fibres dans la cavité de moule, la cavité de moule est fermée et remplie avec l'au moins un deuxième polymère thermoplastique ; et/ou
dans lequel la cavité de moule est exposée à une pression de 1 bar à 2 500 bars durant ledit moulage par injection ou compression et durant ledit refroidissement.

3. Procédé selon l'une quelconque des revendications 1 et 2, dans lequel
l'au moins un deuxième polymère thermoplastique comprend un polymère thermoplastique unique, ou un mélange de polymères,
l'au moins un deuxième polymère thermoplastique comprend un ou plusieurs homopolymères, copolymères et/ou terpolymères, et/ou
l'au moins un deuxième polymère thermoplastique contient en outre, en tant qu'un ou plusieurs additifs, des fibres de carbone hachées et/ou des fibres de verre hachées.

4. Procédé selon l'une quelconque des revendications 1 à 3, dans lequel l'insert renforcé par des fibres comprend une ou plusieurs fibres de polymères, fibres de verre, fibres de carbone, et/ou fibres d'aramide imprégnées avec ledit au moins un premier polymère thermoplastique.

5. Procédé selon l'une quelconque des revendications 1 à 4, dans lequel l'insert renforcé par des fibres est préparé par
mise sous une forme souhaitée d'une ébauche comprenant des fibres imprégnées avec ledit au moins un premier polymère thermoplastique, et
fonte ou dissolution dudit au moins un premier polymère thermoplastique,
dans lequel les couches successives et/ou portions structurelles adjacentes dudit au moins un premier matériau polymère thermoplastique se lient entre elles en formant ainsi l'insert rigide renforcé par des fibres.

6. Procédé selon l'une quelconque des revendications 1 à 5,
dans lequel l'insert renforcé par des fibres est préparé par utilisation d'un enroulement de filaments, d'un placement de bande automatique, et/ou d'un placement de fibres automatique ; et/ou
dans lequel l'insert renforcé par des fibres est préparé par tressage, tissage et/ou tricotage, suivi d'un traitement à la chaleur et/ou avec un solvant, en formant ainsi l'insert rigide renforcé par des fibres.

7. Procédé selon la revendication 6, dans lequel l'insert renforcé par des fibres est préparé par impression 3D de fibres continues, et/ou par un autre traitement pour préparer un insert rigide à partir de fibres continues contenant un matériau préimprégné.

8. Procédé selon l'une quelconque des revendications 1 à 7, dans lequel les fibres sont au moins aussi longues que la dimension la plus petite de la structure d'implant médical renforcé par des fibres moulé, que l'épaisseur de paroi la plus petite, ou que n'importe quelle autre dimension déterminée par la géométrie de la structure d'implant médical renforcé par des fibres moulé, de préférence les fibres sont au moins aussi longues que la dimension principale la plus grande, le diamètre, ou une autre dimension de la structure d'implant médical renforcé par des fibres moulé.

9. Structure d'implant médical renforcé par des fibres moulé, comprenant
un insert rigide renforcé par des fibres, lequel insert rigide renforcé par des fibres contient des fibres continues qui sont imprégnées avec au moins un premier polymère thermoplastique, et
au moins un deuxième polymère thermoplastique, de façon que l'insert rigide renforcé par des fibres soit au moins partiellement recouvert par surmoulage par l'au moins un deuxième polymère thermoplastique dans la structure d'implant médical renforcé par des fibres moulé,
dans laquelle l'au moins un premier polymère thermoplastique et l'au moins un deuxième polymère thermoplastique sont identiques ou différents,
et **caractérisée en ce que** ledit au moins un deuxième polymère thermoplastique contient en outre un ou plusieurs additifs, dans laquelle l'additif est au moins l'un parmi le phosphate de calcium, le bêta-phosphate tricalcique (bêta-TCP), les particules de verre, et un ou plusieurs additifs minéraux.

10. Structure d'implant médical selon la revendication 9, qui a la forme d'une plaque, d'une vis ou d'un clou.

11. Structure d'implant médical selon la revendication 9 ou 10, dans laquelle
l'au moins un deuxième polymère thermoplastique comprend un polymère thermoplastique unique, ou un mélange de polymères,
l'au moins un deuxième polymère thermoplastique comprend un ou plusieurs homopolymères, copolymères et/ou terpolymères, et/ou
l'au moins un deuxième polymère thermoplastique contient en outre, en tant qu'un ou plusieurs additifs, des fibres de carbone hachées et/ou des fibres de verre hachées.

12. Structure d'implant médical selon l'une quelconque des revendications 9 à 11, qui comprend
une ou plusieurs fibres de polymères, fibres de verre, fibres de carbone, et/ou fibres d'aramide imprégnées avec ledit au moins un premier polymère thermoplastique ;
dans laquelle l'insert comprend de multiples couches de fibres continues, chaque couche étant dans une orientation de fibres sélectionnée dans l'insert ; et/ou
dans laquelle l'insert comprend de multiples couches de fibres continues, chaque couche étant dans une orientation de fibres sélectionnée dans l'insert et une construction imprimée en 3D qui est imprimée en 3D sur le dessus de l'insert stratifié.

13. Structure d'implant médical selon l'une quelconque des revendications 9 à 12, dans laquelle l'insert comprend de multiples couches de fibres continues, chaque couche étant dans une orientation de fibres sélectionnée dans l'insert et une construction imprimée en 3D qui est imprimée en 3D sur le cœur de l'insert stratifié.

14. Structure d'implant médical selon l'une quelconque des revendications 9 à 13, dans laquelle les fibres sont au moins aussi longues que la dimension la plus petite de la structure d'implant médical renforcé par des fibres moulé, que l'épaisseur de paroi la plus petite, ou que n'importe quelle autre dimension déterminée par la géométrie de la structure d'implant médical renforcé par des fibres moulé, de préférence les fibres sont au moins aussi longues que la dimension principale la plus grande, le diamètre, ou une autre dimension de la structure d'implant médical renforcé par des fibres moulé.

15. Structure d'implant médical selon l'une quelconque des revendications 9 à 14, dans laquelle l'insert est situé dans le coeur de la structure d'implant médical, à l'intérieur de la structure d'implant médical, ou au moins partiellement sur une surface de la structure d'implant médical.

16. Structure d'implant médical selon l'une quelconque des revendications 9 à 15, qui est produite par le procédé de l'une quelconque des revendications 1 à 8.

17. Implant médical comprenant la structure d'implant médical telle que revendiquée dans l'une quelconque des revendications 9 à 16.
